# EUROPEAN PATENT SPECIFICATION

(11) **EP 2 717 695 B1**
(45) Date of publication and mention of the grant of the patent: **02.11.2016**
(21) Application number: 12796892.3
(22) Date of filing: 04.06.2012
(51) Int. Cl.: A61K 31/4045, A23K 20/168, A23K 50/40, A61P 25/28

(54) **METHODS FOR REDUCING NEURODEGENERATION**
VERFAHREN ZUR VERRINGERUNG EINER NEURODEGENERATION
PROCÉDÉS POUR LA RÉDUCTION DE LA NEURODÉGÉNÉRESCENCE

(30) Priority: 08.06.2011 US 201161520332 P
(43) Date of publication of application: 16.04.2014
(73) Proprietor: Nestec S.A., 1800 Vevey (CH)
(72) Inventor: MIDDLETON, Rondo, Paul, Creve Coeur, Missouri 63141 (US); ZANGHI, Brian, Michael, Gowanda, New York 14070 (US)
(74) Representative: Rupp, Christian
(86) International application number: PCT/US2012/040658
(87) International publication number: WO 2012/170322

(56) References cited:
- US-A1- 2005 164 911
- US-A1- 2011 015 117
- Daniel P Cardinali ET AL: "Clinical aspects of melatonin intervention in Alzheimer's disease progression", Current neuropharmacology, 1 September 2010 (2010-09-01), pages 218-227, XP055116467, United Arab Emirates DOI: 10.2174/157015910792246209 Retrieved from the Internet: URL:http://www.ncbi.nlm.nih.gov/pubmed/213 58972
- YANCHUN GUO ET AL: "Melatonin protects N2a against ischemia/reperfusion injury through autophagy enhancement", JOURNAL OF HUAZHONG UNIVERSITY OF SCIENCE AND TECHNOLOGY, vol. 30, no. 1, 1 February 2010 (2010-02-01), pages 1-7, XP055116460, ISSN: 1672-0733, DOI: 10.1007/s11596-010-0101-9
- CHI HYUN KIM ET AL: "Melatonin-induced autophagy is associated with degradation of MyoD protein in C2C12 myoblast cells", JOURNAL OF PINEAL RESEARCH, vol. 53, no. 3, 14 May 2012 (2012-05-14), pages 289-297, XP055116483, ISSN: 0742-3098, DOI: 10.1111/j.1600-079X.2012.00998.x
- POHANKA: 'Alzheimer's disease and related neurodegenerative disorders: implication and counteracting of melatonin.' APPL BIOMED. vol. 9, 07 April 2011, pages 185 - 196, XP055106041
- KIMBALL ET AL.: 'Melatonin Represses Oxidative Stress-Induced Activation of the MAP Kinase I and mTOR Signaling Pathways in H411E Hepatoma Cells Through Inhibition of Ras.' J PINEAL RES. vol. 44, no. 4, 2008, pages 379 - 386, XP055116399
- ANISIMOV ET AL.: 'Metformin extends life span of HER-2/neu transgenic mice and in combination with melatonin inhibits growth of transplantable tumors in vivo.' CELL CYCLE vol. 9, 01 January 2010, pages 188 - 197, XP002660008

## Description

### CROSS REFERENCE TO RELATED APPLICATIONS

This application claims priority to U.S. Provisional Application Serial No. 61/520332.

### BACKGROUND OF THE INVENTION

### Field of the Invention

The invention relates generally to methods for reducing neurodegeneration and particularly to methods for using melatonin for reducing neurodegeneration in canines.

### Description of Related Art

Neurodegeneration is associated with several severe and lifethreating diseases in animals, *e.g*., Alzheimer's disease, Parkinson's disease, and Huntington's disease. These late-onset diseases are associated with the formation of intracellular aggregates by toxic proteins. An ongoing theory is that the accumulation of these cytoplasmic toxic proteins leads to neurodegeneration because the toxic proteins trigger nerve cell death. In this circumstance, nerve cells have apparently lost the ability to degrade these toxic proteins.

The degradation of cytoplasmic proteins is mediated by a cellular process referred to as macroautophagy, also referred to simply as autophagy. Reducing autophagy in nerve cells leads to neurodegeneration. Autophagy reduction is associated with a deficiency in the atg7 gene or the atg5 gene, genes that produce proteins that play key roles in autophagy.

Several protein kinases have been shown to regulate autophagy. One of the best characterized is the mammalian Target of Rapamycin (mTOR). mTOR is a serine/threonine protein kinase that regulates cell growth, cell proliferation, cell motility, cell survival, protein synthesis, and transcription. mTOR belongs to the phosphatidylinositol 3-kinase-related kinase protein family. mTOR activates the autophagy pathway when in an inactivated or dephosphorylated form. US7052870 discloses methods for regulating the mTOR pathway.

Guo et al (J Huazhong Univ Sci Technol [med Sci], 30(1):2010) discloses a study to explore the mechanisms of the protection of N2a by melatonin in a ischemia/reperfusion model through autophagy.

Methods for modulating autophagy are known. Rapamycin specifically expression of mTOR, which allows the autophagy pathway to be induced. However, longterm rapamycin therapy has adverse side effects, including poor wound healing and immunosuppression.

There is, therefore, a need for new methods for inhibiting the expression of mTOR, increasing autophagy, and reducing neurodegeneration in scanines.

### SUMMARY OF THE INVENTION

It is, therefore, an object of the present invention to provide methods for reducing neurodegeneration in canines.

It is another object of the present invention to provide methods for activating the autophagy pathway in canines.

It is another object of the present invention to provide methods for inhibiting the expression of mTOR in canines.

It is another object of the present invention to provide methods for inhibiting the expression of S6 Kinase in canines.

One or more of these or other objects are achieved by administering melatonin to animals in amounts sufficient for reducing neurodegeneration, activating the autophagy pathway, inhibiting the expression of mTOR, inhibiting the expression of S6 Kinase, or combination thereof. Generally, the melatonin is administered to the canines in amounts of from about 1 ng/kg/day to about 2 mg/kg/day.

Other and further objects, features, and advantages of the present invention will be readily apparent to those skilled in the art.

### DETAILED DESCRIPTION OF THE INVENTION

### Definitions

The term "neurodegeneration" means the progressive loss of neuron structure or function, including neuron death.

The term "melatonin" means 5-methoxy-N-acetyltryptamine and its derivatives or analogs that have substantially the same biological activity as 5-methoxy-N-acetyltryptamine, and their *in vivo* precursors. The term also means any other hormone that has substantially the same biological activity as 5-methoxy-N-acetyltryptamine on the autophagy pathway.

The term "animal" means any canines that could benefit from a reduction in neurodegeneration.

The term "companion animals" means domesticated caninesanimals such as dogs.

The term "ng/kg" means nanograms per kilogram of an animal's body weight.

The term "mg/kg" means milligrams per kilogram of an animal's body weight.

The term "dietary supplement" means a product that is intended to be ingested in addition to the normal animal diet. Dietary supplements may be in any form, *e.g.,* solid, liquid, gel, tablets, capsules, powder, and the like. Preferably they are provided in convenient dosage forms. In some embodiments they are provided in bulk consumer packages such as bulk powders, liquids, gels, or oils. In other embodiments, supplements are provided in bulk quantities to be included in other food items such as snacks, treats, supplement bars, beverages and the like.

As used herein, ranges are used herein in shorthand, so as to avoid having to list and describe each and every value within the range. Any appropriate value within the range can be selected, where appropriate, as the upper value, lower value, or the terminus of the range.

As used herein, the singular form of a word includes the plural, and vice versa, unless the context clearly dictates otherwise. Thus, the references "a", "an", and "the" are generally inclusive of the plurals of the respective terms. For example, reference to "a compound" or "a method" includes a plurality of such "compounds" or "methods." Similarly, the words "comprise", "comprises", and "comprising" are to be interpreted inclusively rather than exclusively. Likewise the terms "include", "including" and "or" should all be construed to be inclusive, unless such a construction is clearly prohibited from the context.

The terms "comprising" or "including" are intended to include embodiments encompassed by the terms "consisting essentially of" and "consisting of". Similarly, the term "consisting essentially of" is intended to include embodiments encompassed by the term "consisting of".

The methods and compositions and other advances disclosed here are not limited to particular methodology, protocols, and reagents described herein because, as the skilled artisan will appreciate, they may vary. Further, the terminology used herein is for the purpose of describing particular embodiments only, and is not intended to, and does not, limit the scope of that which is disclosed or claimed.

Unless defined otherwise, all technical and scientific terms, terms of art, and acronyms used herein have the meanings commonly understood by one of ordinary skill in the art in the field(s) of the invention, or in the field(s) where the term is used. Although any compositions, methods, articles of manufacture, or other means or materials similar or equivalent to those described herein can be used in the practice of the present invention, the preferred compositions, methods, articles of manufacture, or other means or materials are described herein.

### The Invention

In one aspect, the invention provides methods for reducing neurodegeneration in canines. The methods comprise administering a neurodegeneration reducing amount of melatonin to the canines.

In another aspect, the invention provides methods for activating the autophagy pathway in canines. The methods comprise administering an autophagy pathway activating amount of melatonin to the canines.

In another aspect, the invention provides methods for inhibiting the expression of mTOR in canines. The methods comprise administering a mTOR expression inhibiting amount of melatonin to the canines.

In another aspect, the invention provides methods for inhibiting the expression of S6 Kinase in canines. The methods comprise administering a S6 Kinase expression inhibiting amount of melatonin to the canines.

In another aspect, the invention provides methods for affecting one or more conditions or diseases associated with an increase in expression of mTOR by canines. The methods comprise administering a condition or disease affecting amount of melatonin to the animals. The melatonin can be used to affect any condition or disease associated with an increase in mTOR expression by animals, *e.g*., motor neuron diseases. Generally, melatonin is administered to the animal to decrease mTOR expression and therefore affect the condition of such a disease in a way that benefits the animal, *e.g*., increases the health or wellbeing of the animal, lessens the severity of the condition or disease, or alleviates the symptoms caused by the disease or condition or disease.

The invention is based upon the basic discovery that melatonin is effective for one or more of reducing neurodegeneration, activating the autophagy pathway, inhibiting the expression of mTOR, or inhibiting the expression of S6 Kinase in animals. Although not bound by theory, it is believed that melatonin affects the expression of mTOR and S6 Kinase and that regulating such expression activates the autophagy pathway in neurons and reduces neurodegeneration that results from toxic protein accumulation in the neurons.

For such inventive methods, melatonin is administered to the animal in any amount effective for reducing neurodegeneration, activating the autophagy pathway, inhibiting the expression of mTOR, inhibiting the expression of S6 Kinase, affecting one or more conditions or diseases associated with an increase in expression of mTOR, or combination thereof. The melatonin is administered to the animal in amounts of from about 1 ng/kg/day to about 2 mg/kg/day. Skilled artisans can choose the amount of melatonin to administer to an animal based upon age, size, weight, health, disease state, and the like.

Melatonin is administered using any suitable method, preferably orally. Typically, the melatonin is administered in the form of a capsule, gel, tablet, or other suitable oral dosage form. In a preferred embodiment, the melatonin is administered as part of a comestible composition, preferably a food composition or dietary supplement. In certain embodiments, melatonin is administered as a component of a companion animal food composition, preferably a food composition formulated for canines such as dogs. In the most preferred embodiments, the food composition is formulated for dogs. In other embodiments, the melatonin is administered as a dietary supplement.

For oral administration, the melatonin may be incorporated into, for example, tablets or capsules prepared by conventional means with pharmaceutically acceptable excipients such as binding agents (*e.g*., pregelatinised maize starch, polyvinylpyrrolidone or hydroxypropyl methylcellulose); fillers (*e.g*., lactose, microcrystalline cellulose or calcium hydrogen phosphate); lubricants (*e.g*., magnesium stearate, talc or silica); disintegrants (*e.g*., potato starch or sodium starch glycolate); or wetting agents (*e.g*., sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (*e.g*., sorbitol syrup, cellulose derivatives or hydrogenated edible fats); emulsifying agents (*e.g*., lecithin or acacia); non-aqueous vehicles (*e.g*., ationd oil, oily esters, ethyl alcohol or fractionated vegetable oils); and preservatives (*e.g*., methyl or propyl-p-hydroxybenzoates or sorbic acid). The preparations may also contain buffer salts, flavoring, coloring and sweetening agents as appropriate. Preparations for oral administration may be suitably formulated to give controlled release of the melatonin. In a preferred embodiment, the melatonin is incorporated into a tablet or capsule.

The melatonin administered to the animal is any melatonin suitable for administration to an animal. Melatonin is obtained from any suitable source, synthetic or natural. A preferred source of melatonin is synthetic melatonin, *e.g*., in tablet, capsule, and sublingual tablet forms. Other sources include melatonin from natural sources such as algae, fungi, and plants, *e.g*., seeds and walnuts. Melatonin may be derived from a blend of melatonin sources, whether natural or synthetic. For comestible compositions, the melatonin is typically derived from algae or other natural ingredient that is included as a component of the comestible composition, *e.g*., companion animal food composition.

In another aspect, the invention provides packages useful for containing the melatonin of the invention. The package comprises at least one material suitable for containing melatonin and a label affixed to the material containing a word or words, picture, design, acronym, slogan, phrase, or other device, or combination thereof, that indicates that the package contains a compound useful reducing neurodegeneration, activating the autophagy pathway, inhibiting the expression of mTOR, inhibiting the expression of S6 Kinase, affecting one or more conditions or diseases associated with an increase in expression of mTOR by animals, or combination thereof. Typically, such device comprises the words "reduced neurodegeneration" or "activates autophagy" or "inhibits mTOR expression" or "inhibiting the expression of S6 Kinase", or "affects one or more conditions or diseases associated with an increase in expression of mTOR" or "retards aging" or an equivalent expression printed on the material. Any package configuration and packaging material suitable for containing melatonin are useful in the invention, *e.g*., a bag, box, sachet, bottle, can, pouch, and the like manufactured from paper, plastic, foil, metal, and the like. In preferred embodiments, the package further comprises melatonin. In various embodiments, the package further comprises at least one window that permit the package contents to be viewed without opening the package. In some embodiments, the window is a transparent portion of the packaging material. In others, the window is a missing portion of the packaging material.

In another aspect, the invention discloses methods for inhibiting the expression of mTOR or S6 Kinase in *in vitro* neurons. The methods comprise exposing the neurons to a mTOR expression inhibiting or a S6 Kinase expression inhibiting amount of melatonin. Generally, the neurons are exposed to cell culture media with or without serum that typically contains very low or non-detectible levels of melatonin. Therefore, exposing the neurons to media enriched with synthetic melatonin, or extracted or natural sources of melatonin, inhibit mTOR or S6 Kinase expression, induce autophagy processes, or reduce neurodegeneration.

In a further aspect, the invention discloses methods for inhibiting the expression of mTOR or S6 Kinase in *in vitro* cells. These cells include, but are not limited to, primary cells obtained from whole animals, cells from established cell lines, or cells transformed from one type to another. The methods comprise exposing the cells to a mTOR expression inhibiting or a S6 Kinase expression inhibiting amount of melatonin. Generally, the cells are exposed to cell culture media with or without serum that typically contains very low or non-detectible levels of melatonin. Therefore, exposing the cells to media enriched with synthetic melatonin, or extracted or natural sources of melatonin, inhibit mTOR or S6 Kinase expression, induce autophagy processes, or reduce neurodegeneration.

### EXAMPLES

The invention can be further illustrated by the following examples, although it will be understood that these examples are included merely for purposes of illustration and are not intended to limit the scope of the invention unless otherwise specifically indicated.

### Example 1

Cell Culture procedure: Commercially available Madin Darby Canine Kidney (MDCK) cells were obtained from ATCC as a stock suspension of immortalized cells. MDCK cells were cultured at 37°C in an atmosphere of 95% air/5% CO₂ and Dulbecco's Modified Eagle Medium (DMEM) media containing 10% fetal calf serum and 1% antibiotic/antimicrobial solution (DMEM-loaded). The media was changed and cells passed every 1 to 2 days using a solution containing 0.05% trypsin. For RNA extraction, cells were plated at a seeding density of 50,000 to 60,000 cells/cm² in 35 mm dishes. Twenty-four hours after seeding, media was changed to either control (DMEM-loaded) or test media (DMEM-loaded + 1nM melatonin). Cells were harvested 1, 3, or 5 days after exposure to control or test media. Media was changed in the remaining dishes on the days of cell harvesting.

Total RNA was harvested from MDCK cell lysates using RNAlater RNA stabilization reagent and following the manufacturer's directions (Ambion, Austin, Texas). mRNA transcripts of mTOR, S6 Kinase, and TATA box binding protein genes were analyzed by semi-quantitative qPCR on days 1, 3, and 5 of media treatment. TATA box binding protein gene transcript was used to normalize the expression of mTOR and S6 Kinase, as it is widely used as a "housekeeping" gene.

### Example 2

The procedure in Example 1 was repeated. The data from Example 1 and Example 2 are shown in Table 1.

**Table 1**

| | Trial 1 | | Trial 2 | |
|---|---|---|---|---|
| Treatment | mTOR Induction | S6 Kinase Induction | MOTOR Induction | S6 Kinase Induction |
| Day 1 control media | 1.19 | 1.21 | 1.07 | 1.08 |
| Day 1 control media + 1 nM melatonin | 0.93 | 1.14 | 0.98 | 1.05 |
| Day 3 control media | 1.05 | 0.92 | 1.05 | 0.99 |
| Day 3 control media + 1 nM melatonin | 0.76 | 0.66 | 1.02 | 0.82 |
| Day 5 control media | 1.22 | 1.13 | 1.09 | 0.99 |
| Day 5 control media + 1 nM melatonin | 0.49 | 0.51 | 1.01 | 0.75 |

Referring to Table 1, MDCK cells treated with 1nM melatonin in cell culture media for up to 5 days will induce a 7.4 to 59.5% decrease in the mRNA expression of mTOR compared to untreated control cells. In addition, S6 Kinase, which is a target protein of mTOR and is responsible for activating (phosphorylating) ribosomal proteins to stimulate protein synthesis, was also observed to have a decrease in mRNA expression by 24.2 to 55%. This decrease in mTOR and S6 Kinase expression shows inhibition of mTOR pathway by melatonin. These results show that administering melatonin to animals is useful in animals for reducing neurodegeneration, activating the autophagy pathway, inhibiting the expression of mTOR, increasing the expression of the atg5 gene, increasing the expression of the atg7 genes, affecting one or more conditions or diseases associated with an increase in expression of mTOR, or combination thereof.

In the specification, there have been disclosed typical preferred embodiments of the invention. The scope of the invention is set forth in the claims.

## Claims

1. Neurodegeneration reducing amount of melatonin for use in reducing neurodegeneration in a canine,
wherein the melatonin is administered in amounts of from about 1 ng/kg/day to about 2 mg/kg/day.

2. The neurodegeneration reducing amount of melatonin for use according to claim 1 wherein the melatonin is administered in a comestible composition, such as e.g. a food composition.

3. The neurodegeneration reducing amount of melatonin for use according to claim 2 wherein the melatonin is administered as a dietary supplement.

4. mTOR expression inhibiting amount of melatonin for use in reducing neurodegeneration by inhibiting the expression of mTOR in a canine,
wherein the melatonin is administered in amounts of from about 1 ng/kg/day to about 2 mg/kg/day.

5. The mTOR expression inhibiting amount of melatonin for use according to claim 4 wherein the melatonin is administered in a comestible composition.

6. The mTOR expression inhibiting amount of melatonin for use according to claim 5 wherein the melatonin is administered as a dietary supplement.

7. S6 Kinase expression inhibiting amount of melatonin for use in reducing neurodegeneration by inhibiting the expression of S6 Kinase in a canine,
wherein the melatonin is administered in amounts of from about 1 ng/kg/day to about 2 mg/kg/day.

8. The S6 Kinase expression inhibiting amount of melatonin for use according to claim 7 wherein the melatonin is administered in a comestible composition or as a dietary supplement.

9. The S6 Kinase expression inhibiting amount of melatonin for use according to claim 8 wherein the comestible composition is a food composition.

## Patentansprüche

1. Neurodegeneration reduzierende Menge an Melatonin zur Verwendung beim Reduzieren von Neurodegeneration bei einem Hund,
wobei das Melatonin in Mengen von etwa 1 ng/kg/Tag bis etwa 2 mg/kg/Tag verabreicht wird.

2. Neurodegeneration reduzierende Menge an Melatonin zur Verwendung nach Anspruch 1, wobei das Melatonin in einer Nahrungszusammensetzung verabreicht wird, wie z. B. einer Futterzusammensetzung.

3. Neurodegeneration reduzierende Menge an Melatonin zur Verwendung nach Anspruch 2, wobei das Melatonin als Nahrungsergänzung verabreicht wird.

4. Expression von mTOR unterdrückende Menge an Melatonin zur Verwendung beim Reduzieren von Neurodegeneration durch Unterdrücken der Expression von mTOR bei einem Hund,
wobei das Melatonin in Mengen von etwa 1 ng/kg/Tag bis etwa 2 mg/kg/Tag verabreicht wird.

5. Expression von mTOR unterdrückende Menge an Melatonin zur Verwendung nach Anspruch 4, wobei das Melatonin in einer Nahrungszusammensetzung verabreicht wird.

6. Expression von mTOR unterdrückende Menge an Melatonin zur Verwendung nach Anspruch 5, wobei das Melatonin als Nahrungsergänzung verabreicht wird.

7. Expression von S6-Kinase unterdrückende Menge an Melatonin zur Verwendung beim Reduzieren von Neurodegeneration durch Unterdrücken der Expression von S6-Kinase bei einem Hund,
wobei das Melatonin in Mengen von etwa 1 ng/kg/Tag bis etwa 2 mg/kg/Tag verabreicht wird.

8. Expression von S6-Kinase unterdrückende Menge an Melatonin zur Verwendung nach Anspruch 7, wobei das Melatonin in einer Nahrungszusammensetzung oder als Nahrungsergänzung verabreicht wird.

9. Expression von S6-Kinase unterdrückende Menge an Melatonin zur Verwendung nach Anspruch 8, wobei die Nahrungszusammensetzung eine Futterzusammensetzung ist.

## Revendications

1. Quantité réduisant la neurodégénérescence de mélatonine à utiliser dans la réduction de la neurodégénérescence chez un animal canin,
dans laquelle la mélatonine est administrée en quantités d'environ 1 ng/kg/jour à environ 2 mg/kg/jour.

2. Quantité réduisant la neurodégénérescence de mélatonine à utiliser selon la revendication 1, dans laquelle la mélatonine est administrée dans une composition comestible, telle que par ex. une composition comestible.

3. Quantité réduisant la neurodégénérescence de mélatonine à utiliser selon la revendication 2, dans laquelle la mélatonine est administrée sous forme de complément diététique.

4. Quantité inhibant l'expression de mTOR de mélatonine à utiliser pour réduire la neurodégénérescence par inhibition de l'expression de mTOR chez un animal canin,
dans laquelle la mélatonine est administrée en quantités d'environ 1 ng/kg/jour à environ 2 mg/kg/jour.

5. Quantité inhibant l'expression de mTOR de mélatonine à utiliser selon la revendication 4, dans laquelle la mélatonine est administrée dans une composition comestible.

6. Quantité inhibant l'expression de mTOR de mélatonine à utiliser selon la revendication 5, dans laquelle la mélatonine est administrée sous forme d'un complément diététique.

7. Quantité inhibant l'expression de kinase S6 de mélatonine à utiliser pour réduire la neurodégénérescence par inhibition de l'expression de kinase S6 chez un animal canin,
dans laquelle la mélatonine est administrée en quantités d'environ 1 ng/kg/jour à environ 2 mg/kg/jour.

8. Quantité inhibant l'expression de kinase S6 de mélatonine à utiliser selon la revendication 7, dans laquelle la mélatonine est administrée dans une composition comestible ou sous forme d'un complément diététique.

9. Quantité inhibant l'expression de kinase S6 de mélatonine à utiliser selon la revendication 8, dans laquelle la composition comestible est une composition alimentaire.
